# EUROPÄISCHE PATENTSCHRIFT

(11) **EP 1 702 592 B1**
(45) Veröffentlichungstag und Bekanntmachung des Hinweises auf die Patenterteilung: **11.07.2018**
(21) Anmeldenummer: 06004321.3
(22) Anmeldetag: 03.03.2006
(51) Int. Cl.: A61F 2/82

(54) **System zur Behandlung ausgedehnter obliterierender Erkrankungen eines Gefässes**
System for treatment of a diltated obliterated sick vessel
Système de traitement d'un vaisseau dilaté oblitéré

(30) Priorität: 17.03.2005 DE 102005013221
(43) Veröffentlichungstag der Anmeldung: 20.09.2006
(73) Patentinhaber: BIOTRONIK AG, 8180 Bülach (CH)
(72) Erfinder: Tittelbach, Michael, 90429 Nürnberg (DE)
(74) Vertreter: Keck, Hans-Georg

(56) Entgegenhaltungen:
- EP-A- 0 714 640
- EP-A- 1 419 793
- US-A1- 2002 111 603
- US-A1- 2004 243 224

## Beschreibung

### Technisches Gebiet

Die Erfindung betrifft ein System zur Behandlung ausgedehnter obliterierender Erkrankungen eines Gefäßes.

### Hintergrund der Erfindung und Stand der Technik

Bei einer Vielzahl oblitierender Gefäßerkrankungen ist eine rein systemische Behandlung nicht möglich oder führt nur in einem beschränkten Maße zum Erfolg. So ist es beispielsweise bei akuter Angina pectoris oder gar einem vollständigen Verschluss von Arterien in der Regel notwendig, das Gefäß an der betroffenen Stelle wieder aufzuweiten. Dies kann insbesondere durch perkutane transluminale Angioplastie (PTA, häufig auch als Ballonangioplastie bezeichnet) erfolgen, bei der das Gefäß im betroffenen Gefäßabschnitt aufgeweitet wird.

Zumeist wird es notwendig sein, den aufgeweiteten Gefäßabschnitt durch Implantation eines Stents zu stabilisieren, um einen erneuten Gefäßverschluss durch Obstruktion des Gefäßes zu verhindern. Stents sind in der Regel als Permanentimplantate ausgelegt, d. h. verbleiben dauerhaft im Körper des Patienten und können nach dem Einwachsen in die Gefäßwand oft auch nicht mehr operativ entfernt werden. Es hat sich allerdings gezeigt, dass die dauerhafte Anwesenheit eines derartigen Implantats selbst Ausgangspunkt für mikrobiologische Prozesse sein kann, die zu einem erneuten Verschluss des Gefäßes führt (Restenose). Um dem abzuhelfen, wurde vorgeschlagen, die Stents mit Medikamenten zu beschichten, die den zugrunde liegenden mikrobiologischen Prozessen entgegenwirken oder diese gar verhindern. Die Herstellung medikamentenbeschichteter Implantate erfordert die Entwicklung und Durchführung spezifischer Produktions-, Validierungs-, Qualitätssicherung- und Zulassungsprozeduren. Mit anderen Worten, die Etablierung derartiger medikamentenbeschichteter Systeme ist in Entwicklung und Herstellung zeitaufwändig und vor allem kostenträchtig.

US 2002/111603 A1 offenbart ein System zur Behandlung von Gefäßerkrankungen, umfassend einen Stent, der durch transluminale Angioplastie aufgeweitet wurde, und eine zur Freisetzung eines Wirkstoffes im Gewebe des Gefäßes angepasste Vorrichtung. Bei der Vorrichtung handelt es sich um einen Katheter, der ein Medikamentendepot im Gewebe der Gefäßwand einbringt.

Aus US 6,547,803 B2 ist ein modifizierter Ballonkatheter bekannt, mit dem ein Wirkstoff in das Gewebe eines Gefäßes, insbesondere in die Adventitia der Gefäßwand, injiziert werden kann. Dazu weist der Katheter eine Mikronadel in einem expandierbaren Bereich auf, die in etwa senkrecht nach außen stößt und dabei durch die Gefäßwand dringt, wenn der Ballon inflatiert wird. Die Nadel ist mit einem Wirkstoffdepot verbunden und der Wirkstoff kann so in tiefere Gewebeschichten der Gefäßwand eingebracht werden. Es wurde festgestellt, dass sich der Wirkstoff innerhalb der Gefäßwand durch Diffusion weiter ausbreitet. Somit ist eine medikamentöse Behandlung im Gewebe des Gefäßes über einen sich der Injektionsstelle hinausreichenden Bereich möglich.

Aus der US-Patentanmeldung 2004/0010309 A1 ist ein Verfahren und ein dazugehöriges System zur Bereitstellung einer flüssigen Substanz im umgebenen Gewebe eines Blutgefäßes, insbesondere einer Koronararterie, bekannt. Das System umfasst einen Katheter der in US 6,547,803 B2 beschriebenen Art, mit einer Injektionsnadel, über die die flüssige Substanz in das Gewebe eingebracht wird. Weiterhin umfasst das System einen Stent mit einer für die injizierte flüssige Substanz absorptiven Struktur. Nach dem Verfahren wird die Substanz benachbart zum Stützgerüst in das Gewebe des Gefäßes injiziert, von der absorptiven Struktur des Stützgerüstes absorbiert und nach erfolgter Absorption wieder frei gesetzt. Das Stützgerüst wird vor, während oder nach der Injektion der Substanz implantiert.

Stents können auch aus einem biokorrodierbaren Werkstoff hergestellt werden. Diesem Ansatz liegt die Erkenntnis zugrunde, dass die Stützfunktion des Implantates in der Regel nur für einige Wochen bis wenige Monate aufrechterhalten werden muss, in denen sich der behandelte Gefäßabschnitt zumeist schon weitgehend regeneriert hat. Denkbar wäre es, biokorrodierbare Implantate mit Medikamenten zu beschichten, die etwaigen, durch den operativen Eingriff induzierten mikrobiologischen Prozessen entgegenwirken, die zur Restenose führen könnten. Erkenntnisse zur Medikamentenbeschichtung permanenter Implantate lassen sich jedoch nicht einfach auf biokorrodierbare Implantate übertragen, denn hier treten besondere Schwierigkeiten auf. So hat eine Medikamentenbeschichtung, sei es als reiner Wirkstoff oder eingebettet in eine geeignete Matrix, erheblichen Einfluss auf die korrosiven Prozesse, die zum Abbau des Implantates führen. Umgekehrt beeinflussen die korrosiven Prozesse und die dabei entstehenden Abbauprodukte die Elution und Stabilität des Medikaments. Die Wechselwirkungen sind komplex und lassen sich im Allgemeinen nur tendenziell voraussagen. Produktions-, Validierungs-, Qualitätssicherungs- und Zulassungsprozeduren für einen biokorrodierbaren Stent sind entsprechend aufwendig und mit einem erheblichen Kostenaufwand verbunden.

### Zusammenfassung der Erfindung

Aufgabe der vorliegenden Erfindung ist es, ein System zur Behandlung ausgedehnter obliterierender Erkrankungen eines Gefäßes bereitzustellen, das einerseits auf Stents aus biokorrodierbaren Werkstoffen zurückgreift und andererseits eine medikamentöse Behandlung des erkrankten Gefäßes ermöglicht. Dabei soll beiden Aspekten der Behandlung soweit Rechnung getragen werden, dass störende Wechselwirkungen vermieden oder zumindest weitgehend unterdrückt werden.

Das erfindungsgemäße System zur Behandlung ausgedehnter obliterierender Erkrankungen eines Gefäßes, in dem ein Schweregrad der Erkrankung in verschiedenen Gefäßabschnitten des Gefäßes differiert, löst diese Aufgabe.

Das System umfasst:
- ein oder mehrere biokorrodierbare Stents, die voneinander beabstandet in Gefäßabschnitten des erkrankten Gefäßes angeordnet sind, die durch transluminale Angioplastie aufgeweitet wurden, und
- zur Freisetzung eines Wirkstoffs im Lumen oder im Gewebe des Gefäßes angepasstes angepasstes Implantat, das in der Gefäßwand verankert ist und zur Freisetzung des Wirkstoffs ein Wirkstoffdepot aufweist, das mit einer Mikrokanüle verbunden ist, die beim Platzieren des Implantats durch die Gefäßwand dringt und dort die Freisetzung des Wirkstoffs durch Diffusion ermöglicht.

Der Erfindung liegt die Erkenntnis zugrunde, dass eine Entflechtung der Anforderung an die Wirkstoffverteilung im Lumen oder Gewebe des erkrankten Gefäßes, insbesondere auch in die Tiefe des vaskulären Gewebes, sowie an eine exakte, eventuell an die individuellen Patientenbedürfhisse angepasste Dosierung, von den Anforderung an die mechanischen Eigenschaften der biokorrosiven Stents (z. B. Degradationsverhalten, Seitenzugänglichkeit, geo-metrische Optimierung auf maximale Stützwirkung bei minimaler Gefäßwandabdeckung durch alloplastisches Material) insgesamt zu einer Verbesserung der Behandlung und damit Steigerung des Behandlungserfolgs führt. Die konsequente Trennung zwischen dem korrodierenden Implantat und der Vorrichtung für Wirkstoffe ermöglicht auch eine deutliche Vereinfachung der Produktions-, Validierungs-, Qualitätssicherungs- und Zulassungsprozeduren der biokorrosiven Stents, da eine medikamentöse Beschichtung der Stents nicht notwendig ist. Unerwünschte Wechselwirkungen zwischen dem Wirkstoff bzw. einer den Wirkstoff aufnehmenden Matrix und den korrosiven Prozessen beim Abbau des Stents bzw. den Abbauprodukten der Degradation können vermieden oder zumindest deutlich gemindert werden.

Nach einer bevorzugten Ausführungsform der Erfindung besteht der Stent aus einer biokorrosiven Metalllegierung, insbesondere einer biokorrosiven Magnesiumlegierung. Die biokorrosive Magnesiumlegierung ist weiterhin vorzugsweise eine Legierung der Zusammensetzung

| | |
|---|---|
| Yttrium | : 3,7 - 5,5 Gew.% |
| Seltene Erden | : 1,5 - 4,4 Gew.% und |
| Rest | : < 1 Gew.%, |

wobei Magnesium den auf 100 Gew.% fehlenden Anteil an der Legierung einnimmt. Stents aus vorgenannten Werkstoffen zeichnen sich durch ihre günstigen mechanischen Eigenschaften gegenüber synthetischen oder aus natürlichen Quellen hergestellten biokorrodierbaren Polymeren aus. Weiterhin lassen sich insbesondere biokorrosive Magnesiumlegierungen leicht verarbeiten und die Abbauprodukte der Legierung sind sehr körperverträglich.

Gemäß einer Alternative beinhaltet das Implantat zur Freisetzung des Wirkstoffs im Lumen des erkrankten Gefäßes zusätzliche im Lumen des Gefäßes angeordnete Strukturelemente, die die Freisetzung des Wirkstoffes durch korrosive Prozesse ermöglichen. Dann ist bevorzugt, dass das Implantat so angeordnet ist, dass die Freisetzung des Wirkstoffs in das Lumen des Gefäßes mindestens etwa 1 cm, insbesondere mindestens etwa 5 cm, zum nächstliegenden Stent entfernt erfolgt.

Gemäß einer anderen Alternative beinhaltet das Implantat zur Freisetzung des Wirkstoffs im Gewebe des Gefäßes zusätzlich an der Gefäßwand anliegende Strukturelemente, die die Freisetzung des Wirkstoffs in einem oder mehreren Gefäßabschnitten des zu behandelnden Gefäßes ermöglichen, so dass sich der Wirkstoff durch Diffusion im Bereich des erkrankten Gefäßes ausbreiten kann. Dann ist bevorzugt, dass die Vorrichtung so angeordnet ist, dass die Freisetzung des Wirkstoffs im Gewebe des Gefäßes mindestens etwa 0,2 cm, insbesondere mindestens etwa 0,5 cm, zum nächstliegenden Stent entfernt erfolgt. Hiermit wird berücksichtigt, dass die Freisetzung der Wirkstoffe in relativer Nähe zu den erkrankten Gefäßabschnitten, aber nicht unmittelbar am Stent erfolgen sollte, um Wechselwirkungen zwischen dem freigesetzten Wirkstoff und den Produkten des korrosiven Prozesses beim Abbau des Stents zu vermeiden oder zumindest gering zu halten.

Nicht zum Gegenstand der Erfindung gehört ein Verfahren zur Behandlung ausgedehnter obliterierender Erkrankungen eines Gefäßes, in dem ein Schweregrad der Erkrankung in verschiedenen Gefäßabschnitten des Gefäßes differiert, das die Schritte umfasst:
(a) Bereitstellen einer zur Freisetzung eines Wirkstoffs im Lumen oder im Gewebe des Gefäßes angepassten Vorrichtung,
(b) Aufweiten einzelner Gefäßabschnitte des Gefäßes durch transluminale Angioplastie,
(c) Einsetzen eines oder mehrerer biokorrodierbarer Stents in die durch transluminale Angioplastie aufgeweiteten Gefäßabschnitte, und
(d) Freisetzen des Wirkstoffes mittels der Vorrichtung vor, während oder nach Schritt (c), wobei
   (I) die Freisetzung des Wirkstoffs in das Lumen des Gefäßes in einem Gefäßabschnitt erfolgt, der am weitesten proximal in Blutflussrichtung des Gefäßes angeordnet ist;
      oder wobei
   (II) die Freisetzung des Wirkstoffs im Gewebe des Gefäßes in einem oder mehreren Gefäßabschnitten des zu behandelnden Gefäßes erfolgt, so dass sich der Wirkstoff durch Diffusion im Bereich des erkrankten Gefäßes ausbreiten kann.

Nach dem Verfahren können demnach ausgedehnte obliterierende Erkrankungen eines Gefäßes differenziert nach dem Schweregrad der Erkrankung in einzelnen Gefäßabschnitten behandelt werden. Nur die durch transluminale Angioplastie aufgeweiteten Bereiche werden mit einem Stent zur Vermeidung einer Restenose durch Obstruktion des Gefäßes abgestützt. In den weiteren Gefäßabschnitten erfolgt eine Behandlung nur durch Freisetzung des Wirkstoffs. Das Verfahren eignet sich insbesondere zur Behandlung stark verkalkter Gefäßabschnitte.

Das Verfahren erfolgt vorzugsweise unter Freisetzung des Wirkstoffs in das Gewebe, insbesondere die Adventitia des Gefäßes.

Das Verfahren und das System eignen sich insbesondere zur Freisetzung von Wirkstoffen im Zusammenspiel mit biokorrosiven Stents aus einer Magnesiumlegierung, insbesondere der obig angegebenen bevorzugten Zusammensetzung. Besonders bevorzugt sind dabei Wirkstoffe, die im alkalischen Milieu nicht stabil sind oder durch Komplexbildung mit Magnesiumionen deaktiviert werden. Vorzugsweise sind in diesem Zusammenhang die Wirkstoffe Paclitaxel, Sirolimus und Pimecrolimus genannt.

Ferner ist bevorzugt, wenn die Vorrichtung nach dem Freisetzen der Wirkstoffe entfernt wird.

### Kurze Beschreibung der Zeichnungen

- Fig. 1: zeigt einen Ausschnitt aus einem Blutgefäß, enthaltend ein nicht zum Gegenstand der Patentanmeldung gehörenden Variante; und
- Fig. 2: zeigt einen Ausschnitt aus einem Blutgefäß, enthaltend das erfindungsgemäße System.

### Detaillierte Beschreibung mit Ausführungsbeispielen

Die Erfindung betrifft ein System und ein Verfahren zur Behandlung ausgedehnter obliterierender Erkrankungen eines Gefäßes, in dem ein Schweregrad der Erkrankung in verschiedenen Gefäßabschnitten des Gefäßes differiert. Das erfindungsgemäße System wurde bereits vorangehend skizziert und umfasst eine bestimmte Anordnung ein oder mehrerer biokorrodierbarer Stents im Gefäß und eine zur Freisetzung eines Wirkstoffs im Lumen oder im Gewebe des Gefäßes in bestimmter Weise angepasste Vorrichtung.

Unter "Obliteration" wird vorliegend ein Lichtungsverschluss eines Gefäßes, insbesondere durch entzündliche Prozesse (Pleuritis, Perikarditis, Endangiitis), Neoplasma oder Thrombus, verstanden.

"Obliterierende Gefäßkrankheiten" (oder auch Verschlusskrankheiten) betreffen im Sinne der Erfindung:
(i) arteriell: Arteriosklerose, diabetische Angiopathie, entzündliche Gefäßkrankheiten (Arteriitis, Endangiitis), Thrombosen, Embolien
   a) im Extremitätenbereich: periphere arterielle Verschlusskrankheit
   b) viszeral (z.B. Karotisverschluss, koronare Herzkrankheit, Nierenarterienstenose, Verschlüsse größerer Aortenäste); und
(ii) venös: tiefe Venenthrombose, Thrombophlebitis, Phlebitis.

Unter "Gefäß" im Sinne der Erfindung wird die Gesamtheit der arteriellen und venösen Blutgefäße einschließlich der Gefäße der Endstrombahn, die zusammen mit dem Herzen eine funktionelle Einheit bilden, verstanden. Ein solches Gefäß weist ein inneres Lumen auf, durch das das Blut strömt. Das Gefäß wird geometrisch durch seine Gefäßwand definiert, die sich von innen ausgehend von der Intima bis hin zur Adventitia erstreckt.

Unter "ausgedehnt" wird vorliegend, ein längerer Abschnitt des Gefäßes verstanden, der in mindestens zwei Gefäßabschnitte mit unterschiedlichem Schweregrad der obliterierenden Erkrankung gegliedert ist. Dabei ist wenigstens ein Gefäßabschnitt so stark geschädigt, dass eine transluminale Angioplastie zur Aufweitung indiziert ist, und wenigstens ein Gefäßabschnitt vorhanden, in dem dies nicht notwendig ist. Ein "Schweregrad der Erkrankung" im erfindungsgemäßen Sinne stellt demnach zunächst darauf ab, ob der jeweils erkrankte Gefäßabschnitt durch transluminale Angioplastie zur Wiederherstellung seiner Funktionalität aufgeweitet werden muss, oder eben nicht. Zur Einstellung einer Dosierung des freizusetzenden Wirkstoffs kann weiterhin der Schweregrad der Erkrankung differenzierter einfließen, d. h. der Umfang der durch die Erkrankung erfolgten krankhaften Veränderungen im Gefäß kann bei der Einstellung der Dosierung des Wirkstoffs berücksichtigt werden. Vorzugsweise erstreckt sich eine ausgedehnte obliterierende Erkrankung eines Gefäßes im erfindungsgemäßen Sinne über eine Länge des Gefäßes im Bereich von 5 bis 70 cm, insbesondere im Bereich von 10 bis 50 cm.

"Transluminale Angioplastie" beinhaltet vorliegend eine geschlossene perkutane (= perkutane transluminale) Aufdehnung von Gefäßen mit Hilfe koaxialer Katheter oder - heute meist - mittels Ballonkatheters, evtl. als Zusatzmaßnahme bei einer Gefäßoperation. Dies umfasst auch eine perkutane transluminale koronare Angioplastie (PTCA) zur Dehnung von Stenosen der Herzkranzgefäße (= Koronarangioplastie). Die Angioplastie ist neuerdings auch mit Laser- und diversen anderen Techniken möglich.

Unter einer "zur Freisetzung eines Wirkstoffs im Lumen oder im Gewebe des Gefäßes angepassten Vorrichtung" im erfindungsgemäßen Sinne wird eine Vorrichtung verstanden, mit der ein oder mehrere Wirkstoffe aktiv oder passiv im Lumen oder Gewebe des Gefäßes freigesetzt werden können. Aktive Vorrichtungen umfassen Aktuatoren, deren Betätigung den Wirkstoff zum Ort der Freisetzung befördert. Passive Vorrichtungen beinhalten Strukturelemente, die eine Freisetzung der Wirkstoffe durch Diffusion oder in Folge korrosiver Prozesse ermöglichen.

Eine nicht zum Gegenstand der Patentanmeldung gehörende aktive Vorrichtung ist ein zur Freisetzung von Wirkstoffen hergerichteter Ballonkatheter. Ein derartiger Ballonkatheter kann beispielsweise mit dem Wirkstoff beschichtet sein, oder ist so ausgebildet, dass er bei Inflation des Ballons den Wirkstoff aus einem Depot an den gewünschten Ort der Freisetzung befördert. Besonders bevorzugt ist ein Ballonkatheter mit einer Mikronadel, wie er im US-Patent 6,547,803 B2 und in der US-Patentanmeldung 2004/0010309 A1 beschrieben ist. Der Inhalt der beiden Dokumente wird hiermit vollumfänglich durch Verweis einbezogen.

Eine zum Gegenstand dieser Patentanmeldung gehörende passive Vorrichtung kann vorzugsweise als Implantat ausgebildet sein, das in der Gefäßwand verankert wird. Das Implantat beinhalte Strukturelemente, die eine Freisetzung des Wirkstoffs durch Diffusion oder in Folge korrosiver Prozesse ermöglichen. Beispielsweise umfassen diese Strukturelemente flächige Abschnitte mit Vertiefungen, Kammern oder dergleichen, in die der Wirkstoff in einer zur Applikation geeigneten Darreichungsform eingebettet ist. Wenn die flächigen Abschnitte im Lumen des Gefäßes angeordnet sind, wird durch das vorbeiströmende Blut der Wirkstoff herausgelöst bzw. mitgerissen. Liegen die flächigen Abschnitte an der Gefäßwand an, so sind Diffusionsprozesse eher dominierend und der Wirkstoff kann in das Gewebe der Gefäßwand eindringen. Auch die passiven Vorrichtungen können dazu ausgelegt sein, den Wirkstoff in tiefer liegende Gewebsabschnitte der Gefäßwand einzubringen. So beispielsweise durch ein Wirkstoffdepot, das mit einer Mikrokanüle verbunden ist, die beim Platzieren der Vorrichtung durch die Gefäßwand dringt. Aus dem Depot kann dann der Wirkstoff durch Diffusion freigesetzt werden. Vorzugsweise bestehen die passiven Vorrichtungen aus einem biokorrosiven Werkstoff.

Ein "Wirkstoff" im Sinne der Erfindung ist ein pflanzlicher, tierischer oder synthetisierter Stoff, der in geeigneter Dosierung als Therapeutika zur Beeinflussung von Zuständen oder Funktionen des Körpers, als Ersatz für natürlich vom menschlichen oder tierischen Körper erzeugte Wirkstoffe oder Körperflüssigkeiten sowie zur Beseitigung oder zum Unschädlichmachen von Krankheitserregern, Parasiten oder körperfremden Stoffen dient. Die eingesetzten Wirkstoffe sind demnach Arzneimittel und dienen insbesondere der Vorbeugung und Behandlung obliterierender Erkrankungen des Gefäßes. Die zu Zwecken der Freisetzung im Gewebe des Gefäßes verwendeten Wirkstoffe sind vorzugsweise lipophil, da sich lipophile Wirkstoffe besonders gleichmäßig durch Diffusion im Gewebe verteilen. Mit dem System und dem dazugehörigen Verfahren können auch mehrere Wirkstoffe gleichzeitig oder zeitlich versetzt verabreicht werden.

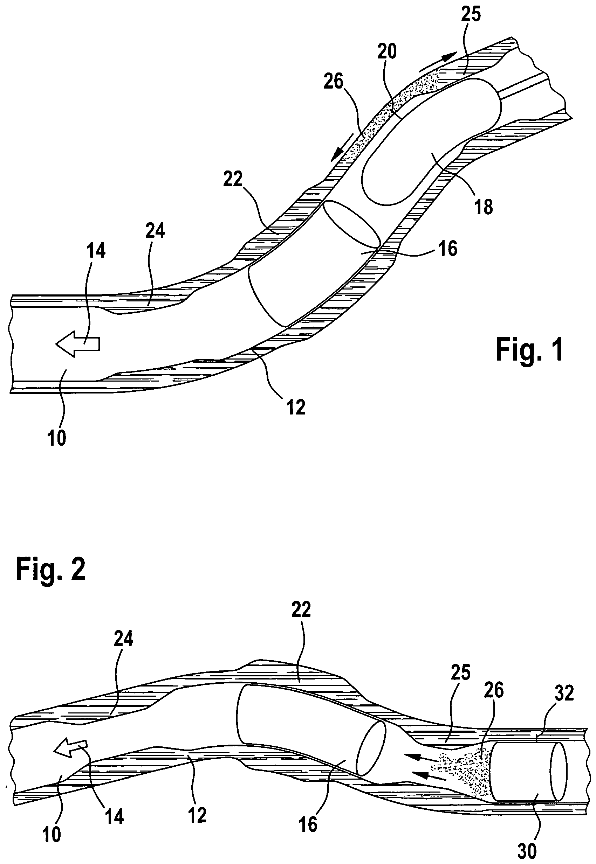

Figur 1 zeigt - stark schematisiert - einen Ausschnitt aus einem Blutgefäß 10 in einem Halbschnitt durch seine Gefäßwand 12. Eine Strömungsrichtung des Blutes im Gefäß 10 wird durch den Pfeil 14 angedeutet.

Das System zur Behandlung dieses Gefäßes 10 umfasst einen biokorrodierbaren Stent 16 und eine Vorrichtung zur Wirkstofffreisetzung in Form eines Ballonkatheters 18 mit einer Mikronadel 20. Diese Vorrichtung ist nicht Gegenstand der Patentanmeldung.

Der biokorrodierbare Stent, der im Gegensatz zu dem hier beschriebenen Ballonkatheter zusammen mit dem erfindungsgemäßen Implantat zum Gegenstand der Patentanmeldung gehört, besteht aus einer Magnesiumlegierung der Zusammensetzung Yttrium 3,7 - 5,5 Gew.%, Seltene Erde: 1,5 - 4,4 Gew.% und Rest < 1 Gew.%, wobei Magnesium den auf 100 Gew.% fehlenden Anteil an der Legierung einnimmt. Der Stent 16 ist in einem Gefäßabschnitt 22 des Gefäßes 10 angeordnet, der zunächst in Folge einer Stenose verengt war und durch transluminale Angioplastie aufgeweitet wurde. Der Stent 16 kann in bekannter Weise mit einem Applikationssystem, z. B. einem Ballonkatheter, an den Implantationsort verbracht werden und dort durch Expansion seiner die Gefäßwand 12 stützenden Funktion im Bereich des Gefäßabschnittes 22 nachkommen.

Zwei weitere Abschnitte 24, 25 des Gefäßes 10 sind bereits durch Kalzifizierung verengt; jedoch rechtfertigt das bisherige Ausmaß der Ablagerung noch keine transluminale Angioplastie und nachfolgende Implantation eines weiteren Stents in diesen Abschnitten 24, 25. Es können jedoch noch weitere Stents im zu behandelnden Gefäß angeordnet sein.

Der nicht zum Gegenstand der Patentanmeldung gehörende Ballonkatheter 18 injiziert über seine Mikronadel 20 einen Wirkstoff 26 in die Gefäßwand 12, wenn er inflatiert wird. Die Freisetzung erfolgt etwa in einem Bereich von 3 bis 5 cm vor dem Stent 16. Der in der Gefäßwand 12, insbesondere der Adventitia freigesetzte Wirkstoff 26 verteilt sich durch Diffusion in der Gefäßwand 12 in longitudinaler Richtung (angedeutet durch die beiden kleinen Pfeile) als auch im Umfangsrichtung des Gefäßes (hier nicht dargestellt). Demnach trifft der Wirkstoff 26 in distaler Blutflussrichtung zunächst auf den Gefäßabschnitt 22 im Bereich des Stents 16 und in proximaler Blutflussrichtung auf den Abschnitt 25 und kann dort jeweils seine pharmakologische Wirkung entfalten.

Eine Menge des injizierten Wirkstoffes hängt selbstverständlich von den Eigenschaften des Wirkstoffes selbst (z. B. Löslichkeit, Abbaugeschwindigkeit im Körper, Diffusionsgeschwindigkeit) und seinem Wirkmechanismus im Organismus ab. Die Wirkstoffmenge wird aber so vorzugeben sein, dass noch eine hinreichende Wirkstoffkonzentration auch im Gefäßabschnitt 24 gegeben ist.

Nach der Injektion des Wirkstoffs 26 wird der Ballonkatheter 18 deflatiert und wieder dem Blutgefäß 10 entnommen. Der Stent 16 verbleibt im Körper, wird jedoch allmählich durch korrosive Prozesse abgebaut.

Figur 2 zeigt ein durch eine ausgedehnte obliterierende Erkrankung geschädigtes Gefäß 10, das in wesentlichen Zügen dem aus Figur 1 gleicht. Insoweit wird auf vorangehende Ausführungen verwiesen und es werden gleiche Bezugszeichen für gleiche oder sehr ähnliche Merkmale verwendet.

Das in Figur 2 dargestellte System gemäß dieser Patentanmeldung unterscheidet sich von dem in Figur 1 dargestellten System dadurch, dass die Vorrichtung zur Freisetzung des Wirkstoffs 26 ein Implantat 30 ist. Das Implantat 30 ist rohrförmiger Kontur und wird vom Blut durchströmt. In seinem Innern weist das Implantat 30 Bauelemente auf, an denen der Wirkstoff 26 in reiner Form oder eingebettet in eine geeignete Matrix gebunden ist, und die so ausgelegt sind, dass das vorbeiströmende Blut den Wirkstoff 26 lösen oder mitreißen kann. Das Implantat ist in einem Gefäßabschnitt 32 angeordnet, der am weitesten proximal in Blutflussrichtung des erkrankten Teils des Gefäßes 10 angeordnet ist. Das Implantat 30 ist vorzugsweise aus einem biodegradierbaren Material geformt, so dass eine nachträgliche operative Entfernung entfallen kann.

## Patentansprüche

1. System zur Behandlung ausgedehnter obliterierender Erkrankungen eines Gefäßes, in dem ein Schweregrad der Erkrankung in verschiedenen Gefäßabschnitten des Gefäßes differiert, umfassend:
- ein biokorrodierbarer Stent (16) oder mehrere - voneinander beabstandete - biokorrodierbare Stents, die in Gefäßabschnitten (22) des erkrankten Gefäßes (10) anordenbar sind, die durch transluminale Angioplastie aufgeweitet wurden, und
- ein zur Freisetzung eines Wirkstoffs (26) angepasstes Implantat (30), das in der Gefäßwand (12) verankerbar ist und zur Freisetzung des Wirkstoffs ein Wirkstoffdepot aufweist, das mit einer Mikrokanüle verbunden ist, die beim Platzieren des Implantats (30) durch die Gefäßwand (12) dringt und dort die Freisetzung des Wirkstoffs durch Diffusion ermöglicht.

2. System nach Anspruch 1, **dadurch gekennzeichnet dass** das Implantat zur Freisetzung des Wirkstoffes im Lumen des erkrankten Gefäßes zusätzliche im Lumen des Gefäßes angeordnete Strukturelemente beinhaltet, die die Freisetzung des Wirkstoffes durch korrosive Prozesse ermöglichen.

3. System nach Anspruch 2, bei dem das Implantat (30) so angeordnet ist, dass die Freisetzung des Wirkstoffs (26) in das Lumen des Gefäßes (10) mindestens etwa 1 cm, insbesondere mindestens etwa 5 cm, zum nächstliegenden Stent (16) entfernt erfolgt.

4. System nach Anspruch 1, **dadurch gekennzeichnet, dass** das Implantat zur Freisetzung des Wirkstoffs im Gewebe des Gefäßes zusätzlich an der Gefäßwand anliegende Strukturelemente (20) beinhaltet, die die Freisetzung des Wirkstoffs in einem oder mehreren Gefäßabschnitten (32) des zu behandelnden Gefäßes ermöglichen, so dass sich der Wirkstoff durch Diffusion im Bereich des erkrankten Gefäßes ausbreiten kann.

5. System nach Anspruch 4, bei dem das Implantat (30) so angeordnet ist, dass die Freisetzung des Wirkstoffs (26) im Gewebe des Gefäßes (10) mindestens etwa 0,2 cm, insbesondere mindestens etwa 0,5 cm, zum nächstliegenden Stent (16) entfernt erfolgt.

6. System nach einem der Ansprüche 1 bis 5, bei dem der Wirkstoff (26) Paclitaxel, Sirolimus und Pimecrolimus umfasst.

7. System nach einem der Ansprüche 1 oder 5, **dadurch gekennzeichnet, dass** das Implantat (30) eine rohrförmige Kontur, die von Blut durchströmbar ist, aufweist.

8. System nach einem der Ansprüche 1, 2 oder 5, **dadurch gekennzeichnet, dass** die Strukturelemente flächige Abschnitte, vorzugsweise mit Vertiefungen oder Kammern oder dergleichen, in die der Wirkstoff (26) in einer zur Applikation geeigneten Darreichungsform eingebettet ist, aufweisen.

9. System nach Anspruch 1, bei dem der oder die Stents (16) aus einer biokorrosiven Metalllegierung bestehen.

10. System nach Anspruch 9, bei dem die biokorrosive Magnesiumlegierung eine Legierung der Zusammensetzung
Yttrium: 3,7 - 5,5 Gew.-%,
Seltene Erden: 1,5 - 4,4 Gew.-% und
Rest: < 1 Gew.-%,
ist, wobei Magnesium den auf 100 Gew.% fehlenden Anteil an der Legierung einnimmt.

## Claims

1. A system for treating extensive obliterative vascular diseases, in which the severity of the disease differs in different sections of the vessel, comprising:
- a biocorrodible stent (16), or a plurality of biocorrodible stents-spaced apart from one another-which can be arranged in vascular sections (22) of the diseased vessel (10) that have been dilated by way of transluminal angioplasty, and
- an implant (30) adapted for release of an active ingredient (26), which can be anchored in the vascular wall (12) and comprises an active ingredient depot for releasing the active ingredient, which is connected to a microcannula that, during placement of the implant (30), penetrates the vascular wall (12) and allows the active ingredient to be released there by way of diffusion.

2. The system according to claim 1, **characterized in that** the implant for release of the active ingredient in the lumen of the diseased vessel comprises additional structural elements arranged in the lumen of the vessel which allow the active ingredient to be released by way of corrosive processes.

3. The system according to claim 2, wherein the implant (30) is arranged so that the active ingredient (26) is released into the lumen of the vessel (10) at least approximately 1 cm, and in particular at least approximately 5 cm, away from the closest stent (16).

4. The system according to claim 1, **characterized in that** the implant for release of the active ingredient in the lumen of the diseased vessel comprises additional structural elements (20) resting against the vascular wall which allow the active ingredient to be released in one or more vascular sections (32) of the vessel to be treated, so that the active ingredient can spread in the area of the diseased vessel by way of diffusion.

5. The system according to claim 4, wherein the implant (30) is arranged so that the active ingredient (26) is released in the tissue of the vessel (10) at least approximately 0.2 cm, and in particular at least approximately 0.5 cm, away from the closest stent (16).

6. The system according to any one of claims 1 to 5, wherein the active ingredient (26) comprises paclitaxel, sirolimus and pimecrolimus.

7. The system according to claim 1 or 5, **characterized in that** the implant (30) has a tubular contour through which blood can flow.

8. The system according to claim 1, 2 or 5, **characterized in that** the structural elements include planar sections, preferably having indentations or pockets or the like, in which the active ingredient (26) is embedded in a form of administration suitable for application.

9. The system according to claim 1, wherein the stent or stents (16) is or are made of a biocorrosive metal alloy.

10. The system according to claim 2,
wherein the biocorrodible magnesium alloy is an alloy having the composition yttrium: 3.7 to 5.5 wt.%,
rare earths: 1.5 to 4.4 wt..-% and
remainder: < 1 wt.%,
wherein magnesium accounts for the content in the alloy that is missing to make up 100 wt.%.

## Revendications

1. Système de traitement de maladies d'un vaisseau dilaté oblitéré, dans lequel un degré de sévérité de la maladie diffère dans diverses sections de vaisseau du vaisseau, comprenant :
- un stent biocorrodable (16) ou plusieurs stents biocorrodables, espacés les uns des autres, qui peuvent être agencés dans des sections de vaisseau (22) du vaisseau malade (10), qui ont été élargis par angioplastie transluminale, et
- un implant (30) adapté pour la libération d'une matière active (26) qui peut être ancré dans la paroi de vaisseau (12) et présente un dépôt de matière active pour la libération de la matière active, qui est relié avec une micro canule, traverse la paroi de vaisseau (12) lors de la mise en place de l'implant (30), et y permet la libération de la matière active par diffusion.

2. Système selon la revendication 1, **caractérisé en ce que** l'implant contient des éléments de structure additionnels disposés dans la lumière du vaisseau pour la libération de la matière active dans la lumière du vaisseau malade, qui permettent la libération de la matière active par des processus de corrosion.

3. Système selon la revendication 2, chez lequel l'implant (30) est disposé de telle manière que la libération de la matière active (26) dans la lumière du vaisseau (10) a lieu éloignée d'au moins environ 1 cm, en particulier, d'au moins environ 5 cm par rapport au stent (16) le plus proche.

4. Système selon la revendication 1, **caractérisé en ce que** l'implant contient additionellement des éléments de structure (20) adjacents à la paroi de vaisseau pour la libération de la matière active dans le tissu du vaisseau, qui permettent la libération de la matière active dans un ou plusieurs segments de vaisseau (32) du vaisseau à traiter, de sorte que la matière active peut s'étendre par diffusion dans la région du vaisseau malade.

5. Système selon la revendication 4, chez lequel l'implant (30) est disposé de telle manière que la libération de la matière active (26) dans le tissu du vaisseau (10) a lieu éloignée d'au moins environ 0,2 cm, en particulier, d'au moins environ 0,5 cm par rapport au stent (16) le plus proche.

6. Système selon l'une des revendications 1 à 5, chez lequel la matière active (26) comprend du paclitaxel, du sirolimus et du pimécrolimus.

7. Système selon l'une des revendications 1 à 5, **caractérisé en ce que** l'implant (30) présente une structure en forme de tube qui peut être traversée par du sang.

8. Système selon l'une des revendications 1, 2 ou 5, **caractérisé en ce que** les éléments de structure présentent des portions planes, de préférence, avec des creux ou des chambres ou similaires dans lesquels la matière active (26) est incorporé dans une forme d'administration appropriée pour l'application.

9. Système selon la revendication 1, chez lequel le ou les stents (16) sont constitués d'un alliage métallique biocorrodable.

10. Système selon la revendication 9, chez lequel l'alliage métallique biocorrodable est un alliage de la composition
Yttrium : 3,7 à 5,5 % en poids,
Terres rares : 1, 5 à 4, 4 % en poids, et
reste : < 1 % en poids,
où le magnésium constitue la partie manquante jusqu'à 100 % en poids de l'alliage.
